(19) Europäisches Patentamt
European Patent Office
Office européen des brevets

(11) Veröffentlichungsnummer: **0 234 462**
**B1**

(12) **EUROPÄISCHE PATENTSCHRIFT**

(45) Veröffentlichungstag der Patentschrift: **30.05.90**

(51) Int. Cl.$^5$: **C 07 D 265/30,** A 01 N 43/84

(21) Anmeldenummer: **87102114.3**

(22) Anmeldetag: **14.02.87**

(54) **Salz eines substituierten Morpholins mit Fluoroborsäure und Holzschutzmittel.**

(30) Priorität: **18.02.86 DE 3605007**

(43) Veröffentlichungstag der Anmeldung:
**02.09.87 Patentblatt 87/36**

(45) Bekanntmachung des Hinweises auf die Patenterteilung:
**30.05.90 Patentblatt 90/22**

(84) Benannte Vertragsstaaten:
**AT BE CH DE ES FR GB GR IT LI NL SE**

(56) Entgegenhaltungen:
**EP-A-0 075 814**
**DE-A-2 461 513**
**DE-B-1 173 722**

(73) Patentinhaber: **Dr. Wolman GmbH**
**Dr.-Wolman-Strasse 31-33**
**D-7573 Sinzheim (DE)**

(72) Erfinder: **Goettsche, Reimer, Dr.**
**Waldstrasse 27**
**D-7570 Baden-Baden 19 (DE)**
Erfinder: **Marx, Hans-Norbert**
**Mozartweg 8**
**D-7580 Buehl-Weitenung (DE)**

(74) Vertreter: **Schweiss, Werner, Dr. et al**
**BASF Aktiengesellschaft Patentabteilung Carl-Bosch-Strasse 38**
**D-6700 Ludwigshafen (DE)**

**Die Akte enthält technische Angaben, die nach dem Eingang der Anmeldung eingereicht wurden und die nicht in dieser Patentschrift enthalten sind.**

**EP 0 234 462 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Beschreibung

Die vorliegende Erfindung betrifft das neue Salz des N-Tridecyl-2,6-dimethylmorpholins mit Fluoroborsäure

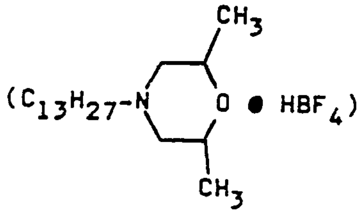

und

seine Anwendung als Fungizid, insbesondere zum Schutz von Holz.

Es ist bekannt, N-Tridecyl-2,6-dimethylmorpholin (Tridemorph) als Fungizid zu verwenden (DE 1 164 152). Die Verbindung zeigt jedoch keine Wirkung gegen Insekten.

Es ist auch bekannt, das Salz des 2-(Methoxycarbonylamino)-benzimidazols mit Fluoroborsäure als Fungizid im Holzschutz zu verwenden (DE—3 138 575.3). Durch die Fluoroborsäure wird ein zusätzlicher fungizider Wirkstoff in das Benzimidazolderivat eingeführt.

Es ist ferner bekannt, wasserlösliche Salze der Fluoroborsäure mit Alkalidichromat im Holz zur Umsetzung zu bringen und dadurch das Holz zu schützen, diese Salze sind als CFB-Salze im Handel.

Die wasserlöslichen Salze der Fluoroborsäure werden im Holz nicht festgelegt (nicht fixiert).

Sie können nur durch Zusatz von Chromaten im Holz über Chrom-Kryolithbildung (z.B. $Na_3CrF_6$) fixiert werden. Diese Fluorfixierung ist jedoch nicht vollkommen, da sich die freien Fluoridionen aus den Fluoroboraten erst bei pH-Werten über pH 7 im Holz bilden und hierzu ein erheblicher Chromatüberschuß notwendig ist. Bei den üblichen Salzgemischen (ca. 33% Alkalidichromat) wird über 50% des Fluors mit Wasser ausgewaschen. Die Salzgemische benötigen für die Fixierung außerdem 2 bis 6 Wochen je nach Klimabedingungen, hierbei kann Chrom (VI) aus dem Holz ausgewaschen werden und dadurch die Umwelt belasten.

Es wurde jetzt gefunden, daß das Salz des N-Tridecyl-2,6-dimethylmorpholins mit Fluoroborsäure ($HBF_4$) eine sehr gute fungizide und insektizide Wirkung hat. Es ist in Wasser unlöslich, aber mit Hilfe von ionischen oder nichtionischen Emulgatoren, gegebenenfalls unter Zusatz von organischen Lösungsmitteln lassen sich Emulsionskonzentrate herstellen, die in Wasser klare Mikroemulsionen bilden. Diese Mikroemulsionen können durch übliche handwerkliche Verfahren (z.B. Streichen, Tauchen, Sprühen) oder großtechnische Verfahren (z.B. Kesseldruckverfahren, Doppelvakuumverfahren) ins Holz eingebracht werden und bewirken einen ausreichenden vorbeugenden Schutz gegen holzzerstörende tierische und pflanzliche Holzzerstörer, auch im Außenbereich, da sie durch Witterungseinflüsse nicht ausgewaschen werden.

Herstellungsbeispiel

Man setzt 30 g technisches N-Tridecyl-2,6-dimethylmorpholin mit 8,8 g Fluoroborsäure in 26,4 g Wasser um und erhält einen wasserunlöslichen pastösen Niederschlag, der abfiltriert wird. Nach dem Trocknen erhält man 38,5 g Salz des N-Tridecyl-2,6-dimethylmorpholins mit Fluoroborsäure mit dem Schmelzpunkt ca. 30°C.

Man kann beispielsweise Tridemorphfluoroborat mit organischen Lösungsmitteln und nichtionischen Emulgatoren mischen und die Mischung in Wasser emulgieren. Es ist auch möglich, Tridemorph in Mischung mit Dimethyl($C_{10}$—$C_{16}$)alkylaminen durch Fluoroborsäure (in äquivalenter Menge zu Tridemorph oder im Überschuß), gegebenenfalls unter Zusatz von substituierten Carbonsäuren, z.B. Milchsäure, Weinsäure, Gluconsäure, Adipinsäure) und/oder Phosphorsäure oder Phosphonsäure zu neutralisieren, so daß die Salze des Dimethylalkylamins emulgierend auf das Tridemorphfluoroborat wirken und mit Wasser wäßrige Mikroemulsionen bilden. Die Dimethylalkylaminsalze können auch in Mischung mit Lösungsmitteln oder nicht-ionischen Emulgatoren dem Tridemorphfluoroborat zugesetzt werden. Durch die eigene fungizide Wirksamkeit der Dimethylalkylamine wird die Holzschutzwirksamkeit der Mischung zusätzlich verbessert. Es ist überraschend, daß das neue Salz in einfacher Weise in Wasser emulgiert werden kann, wobei klare Mikroemulsionen entstehen.

Zur Verbesserung des Korrosionsverhaltens gegenüber Eisen oder Stahl können gegebenenfalls Carbonsäuren, z.B. Isononansäure, Isooctansäure oder p-tertiär-Butylbenzoesäure zugesetzt werden.

Die folgenden Beispiele erläutern Mischungen, die in Wasser zu Mikroemulsionen emulgiert werden können.

### Beispiel 1

20% Tridemorph (Gewichtsprozent)
6% Fluoroborsäure
18% Dipropylenglykol
50% Ethoxyliertes Nonylphenol (9 Mol Äthylenoxid je Mol subst. Phenol)
6% wasser

Eine 10%ige Emulsion der Mischung in Wasser kann z.B. für handwerkliche Verfahren verwendet werden.

### Beispiel 2

20% Tridemorph
31% Dimethyl-$C_{10}$—$C_{16}$-alkylamingemisch
9,0% Milchsäure
10% Fluoroborsäure (Überschuß)
20% Butylglykolacetat
10,0% Wasser

Eine 10%ige Emulsion der Mischung in Wasser kann z.B. für großtechnische oder handwerkliche Verfahren verwendet werden.

Beispiel 3

20% Tridemorph
33% Dimethyl-$C_{16}$-alkylamin
12% Phosphorsäure
6% Fluoroborsäure
20% Propylenglykol
9% Wasser

Eine 1,5%ige Emulsion der Mischung in Wasser kann z.B. für großtechnische Verfahren verwendet werden.

Beispiel 4

20% Tridemorph
30% Dimethyl-$C_{12}$-alkylamin
10% ethoxyliertes Nonylphenol (9 Äthylenoxid je Phenol)
10% Propylenglykol
10% Fluoroborsäure
7% Phosphorsäure
1,3% Isooctansäure
11,7% Wasser

Eine 1 — 10%ige Emulsion der Mischung in Wasser kann für großtechnische oder handwerkliche Verfahren verwendet werden.

Beispiel 5

20% Tridemorph
30% Dimethyl-$C_{14}$-alkylamin
20% Propylenglykol
10% Fluoroborsäure
5% Gluconsäure
15% Wasser

Eine 1 — 10%ige Emulsion der Mischung in Wasser kann für großtechnische oder handwerkliche Verfahren verwendet werden.

Beispiel 6

20% Tridemorph
35% Dimethylalkylamingemisch ($C_{12}$—$C_{16}$)
11,5% Fluoroborsäure
22,0% Milchsäure
11,5% Wasser

Es können auch andere Carbonsäuren, z.B. Zitronensäure, Weinsäure, Adipinsäure verwendet werden. Für die einfache Herstellung der Mischungen empfiehlt sich jedoch die Anwendung von Säuren, die handelsüblich in flüssiger Form vorliegen (z.B. Milschsäure 90%). Solche Mischungen in Form von Lösungen sind durch Mischen der Einzelbestandteile sofort erhältlich. Bei kristallinen Säuren (wie zuvor genannt) sind zur Herstellung von Lösungen lange Rührzeiten erforderlich. Die Fluoroborsäure kann auch in Überschuß vorliegen, durch Diffusion werden dann solche Bereiche des Holzes geschützt, die einer Imprägnierung nicht zugänglich sind (Kernholz).

Es empfiehlt sich je nach Säurezugabe und Anwendungskonzentrationen einen pH-Wert von 2,0—5,0, vorzugsweise 3,0—4,0 in der Mischung einzustellen.

Die Mischungen können durch wasserlösliche Kontrollfarbstoffe eingefärbt werden. Durch wasserunlösliche Farbstoffe, die sich in den Mischungen lösen und mit diesen emulgiert werden, lassen sich witterungsbeständige Anfärbungen des Holzes erzielen.

**Patentansprüche**

1. Salz des N-Tridecyl-2,6-dimethylmorpholins mit Fluoroborsäure.
2. Fungizid, enthaltend das Salz des N-Tridecyl-2,6-dimethylmorpholins mit Fluoroborsäure.
3. Fungizid, enthaltend einen festen oder flüssigen Trägerstoff und das Salz des N-Tridecyl-2,6-dimethylmorpholins mit Fluoroborsäure.
4. Holzschutzmittel, enthaltend das Salz des N-Tridecyl-2,6-dimethylmorpholins mit Fluoroborsäure.
5. Holzschutzmittel, enthaltend einen festen oder flüssigen Trägerstoff und das Salz des N-Tridecyl-2,6-dimethylmorpholins mit Fluoroborsäure.
6. Verfahren zur Bekämpfung von Pilzen, dadurch gekennzeichnet, daß man die Pilze oder das durch Pilzbefall bedrohte Holz mit dem Salz des N-Tridecyl-2,6-dimethylmorpholins mit Fluorborsäure behandelt.
7. Verfahren zur Bekämpfung von Insekten, dadurch gekennzeichnet, daß man die Insekten oder das durch Insekten bedrohte Holz mit dem Salz des N-tridecyl-2,6-dimethylmorpholins mit Fluorborsäure behandelt.
8. Mischung enthaltend das Salz des N-Tridecyl-2,6-dimethylmorpholins mit Fluoroborsäure, das Salz einer Dimethyl-$C_{10}$—$C_{16}$-alkylaminmischung mit einer substituierten Carbonsäure und Propylenglykol.
9. Mikroemulsion, hergestellt durch Emulgieren einer Mischung gemäß dem vorhergehenden Anspruch mit Wasser.

**Revendications**

1. Sel de la N-tridécyl-2,6-diméthylmorpholine et d'acide fluoborique.
2. Fongicide contenant le sel de la N-tridecyl-2,6-diméthylmorpholine et d'acide fluoborique.
3. Fongicide contenant un support solide ou liquide et le sel de la N-tridécyl-2,6-diméthylmorpholine et d'acide fluoborique.
4. Agent de protection du bois contenant le sel de la N-tridécyl-2,6-diméthylmorpholine et d'acide fluoborique.
5. Agent de protection du bois, contenant un support solide ou liquide et le sel de la N-tridécyl-2,6-diméthylmorpholine et d'acide fluoborique.
6. Procédé de lutte contre les champignons, caractérisé par le fait que l'on traite les champignons ou le bois menacé par l'attaque par les champignons avec le sel de la N-tridécyl-2,6-diméthylmorpholine et d'acide fluoborique.
7. Procéde de lutte contre les insectes, caractérisé par le fait que l'on traite les insectes ou le bois menacé par les insectes avec le sel de la N-tridécyl-2,6-diméthylmorpholine et d'acide fluoborique.
8. Mélange contenant le sel de la N-tridécyl-2,6-diméthylmorpholine et d'acide fluoborique, le sel d'un mélange diméthyl-alkyl($C_{10}$—$C_{16}$) amine et d'un acide carboxylique substitué et du propylène-glycol.

9. Micro-émulsion, préparée par émulsification d'un mélange selon la revendication précédente dans de l'eau.

**Claims**

1. The salt of N-tridecyl-2,6-dimethylmorpholine with fluoboric acid.

2. A fungicide containing the salt of N-tridecyl-2,6-dimethylmorpholine with fluorboric acid.

3. A fungicide containing a solid or liquid carrier and the salt of N-tridecyl-2,6-dimethylmorpholine with fluoboric acid.

4. A wood preservative containing the salt of N-tridecyl-2,6-dimethylmorpholine with fluoboric acid.

5. A wood preservative containing a solid or liquid carrier and the salt of N-tridecyl-2,6-dimethylmorpholine with fluoboric acid.

6. A method of controlling fungi, wherein the fungi of the wood threatened by fungal attack are treated with the sale of N-tridecyl-2,6-dimethylmorpholine with fluoboric acid.

7. A method of controlling insects, wherein the insects or the wood threatened by insects are treated with the salt of N-tridecyl-2,6-dimethylmorpholine with fluoboric acid.

8. A mixture containing the salt of N-tridecyl-2,6-dimethylmorpholine with fluoboric acid, the salt of a dimethyl-$C_{10}$—$C_{16}$-alkylamine mixture with a substituted carboxylic acid, and propylene glycol.

9. A microemulsion prepared by emulsifying a mixture as claimed in the preceding claim with water.